# EUROPEAN PATENT APPLICATION

(11) **EP 2 210 584 A1**
(43) Date of publication of application: **28.07.2010**
(21) Application number: 09151405.9
(22) Date of filing: 27.01.2009
(51) Int. Cl.: A61K 9/00, A61K 9/107, A61K 47/34

(54) **Stable polymeric composition comprising an epothilone and an amphiphilic block copolymer**

(71) Applicant: Bayer Schering Pharma Aktiengesellschaft, 13353 Berlin (DE)
(72) Inventor: Richter, Annett, D-10119 Berlin (DE); Olbrich, Carsten, D-10961 Berlin (DE); Krause, Michael, D-14195 Berlin (DE); Kissel, Thomas, D-79219 Staufen (DE)

(57) **Abstract**

The present invention describes a stable polymeric composition comprising an epothilone and an amphiphilic block copolymer having a hydrophilic poly(alkylene glycol) component and a hydrophobic biodegradable polymer component.

## Description

### Brief Description of the invention

The present invention describes a stable polymeric composition comprising an epothilone and an amphiphilic block copolymer having a hydrophilic poly(alkylene glycol) component and a hydrophobic biodegradable polymer component. The composition is capable to form a micellar dispersion, solubilizing the epothilone, in an aqueous medium, which can be injected into the body undiluted or after further dilution with aqueous media.

### Background of the invention

It is still a challenge to develop a suitable, biocompatible formulation for poorly water soluble drugs.

Epothilones, consisting of a 16-membered lactone ring which is also called a "macrolide" ring, are known to be a class of poorly water soluble anti-cancer drugs e.g. see page 2 of WO 99/39694, wherein it is reported that the macrolide ring is particular labile to degradation and also to decomposition in water and their poor solubility makes it difficult to form pharmaceutical formulations for parenteral administration.

There are several compositions known comprising epothilones e.g. WO 2006/066949 , WO 99/39694 containing Cremophor to facilitate solubility, WO 2005/044198 WO 2005/034264 . One disadvantage of almost all of these formulations is a direct contact of the drug with water during the process of formulation preparation. Since the drug itself is prone to degradation over prolonged periods of storage in aqueous solutions, a second step, mostly freeze drying, is necessary to obtain a stable composition for sale, which is reconstituted in the laboratories of the clinics prior to administration. The freeze drying step per se is stressing the labile drugs and is time consuming and expensive. Thus a process preventing this step would be advantageous in view of industrial applicability as well as in view of preserving the drug.

An alternative to freeze-drying is the production of infusion concentrates wherein the drug is dissolved in organic solvents and/ or solubilizing excipients. All of the compositions discussed above contain further excipients which in some events can cause unwanted side effects and can thus be disadvantageous for the patient receiving the formulation for a therapy e.g. the formulation disclosed in WO 99/39694 is containing Cremophor to facilitate solubility, which can only be used in low concentrations due to its toxic side effects such as hypersensitivity.

Amphiphilic block copolymers are known to form polymeric micelles capable to solubilize hydrophobic drugs e.g. Paclitaxel (X. Zhang et al., Int. J. Pharm. 132(1996) 195-206). The preparation process of this polymeric micelles consists of the formation of a matrix consisting of the amphiphilic polymer and Paclitaxel by evaporation of an organic solvent under a stream of nitrogen at 60°C. Afterwards redispersion of the matrix is carried out by preheating this matrix to 60°C and addition of water at 60°C resulting in the micellar dispersion. Since epothilones are prone to degradation at elevated temperatures this preparation method is unsuitable for this class of compounds.

A drug composition containing Paclitaxel is known from WO 01/87345 comprising in addition a block copolymer whereby the hydrophobic block of the copolymer has been modified to contain an end group, which improves the affinity to the poorly water soluble drug. The modification is reported to directly improve the stability of the micelles and the drug entrapped therein. The process for the production of this composition also includes a freeze drying step as well as direct contact of the drug with water.

WO 01/12718 discloses polymeric compositions comprising an amphiphilic block copolymer having a hydrophilic poly(alkylene glycol) component and a hydrophobic biodegradable polymer component, contained in a poly(ethylene glycol) (PEG) medium. The composition can be stored as a stable liquid formulation and is capable to form polymeric micellar dispersions in an aqueous medium solubilizing hydrophobic drugs. It is shown that comparative compositions containing the above mentioned block copolymers and a hydrophobic drug but no poly(ethylene glycol) failed to form micellar dispersions in an aqueous medium. Therefore the addition of poly(ethylene glycol) is necessary to obtain a stable composition for sale, increasing the amount of excipients used in the formulation again.

Thus there is still the need to provide a suitable formulation for epothilones and especially providing a process for its production diminishing decomposition of the drug. Additionally it would be advantageous, if direct contact of the drug with water as well as the lyophilisation step could be prevented and less excipients were included.

Therefore the task of the invention was to overcome the problem of degradation of the drug during the preparation steps of the composition resulting in a stable formulation. The problem has been solved by providing a composition according to the invention which can be prepared without water contact, prevention of lyophilization and no need of the addition of further excipients like PEG.

### Description of the invention

The invention relates to a stable polymeric composition comprising an amphiphilic AB type diblock copolymer having
a hydrophilic poly(alkylene glycol) component (A) and
an amorphous hydrophobic biodegradable polymer component (B), and an epothilone.

The composition of the present invention is capable to form micellar dispersions solubilizing the epothilone after redispersion with an aqueous medium.

It has been surprisingly found that the composition mentioned above needs not only no poly(ethylene glycol) as a solvent or dispersant as predicted by prior art but also no further excipients for the preparation for maintaining the good redispersion behaviour over storage time.
Thus the lyophilisation step to obtain a formulation for sale can be prevented. The composition is nevertheless stable and well reconstitutionable for use.

In one aspect of the invention the amphiphilic block copolymer is a AB type diblock copolymer comprising
a poly(alkylene glycol) component (A) and
an amorphous hydrophobic biodegradable polymer component (B)

As hydrophilic component (A) the following polymers are suitable: poly(ethylene glycol) (PEG), monomethoxypoly(ethylene glycol) (mPEG), monoethoxypoly(ethylene glycol), monopropoxypoly(ethylene glycol), monobutoxypoly(ethylene glycol), monoformylpoly(ethylene glycol), monoacetylpoly(ethylene glycol), monopropionylpoly(ethylene glycol) und monobutyrypoly(ethylene glycol).
The molecular weight of the hydrophilic component is in the range of 1000 to 5000 Daltons, and more preferably within the range of 2000 to 5000.

As amorphous hydrophobic biodegradable polymer component (B) the following polymers are suitable: poly(D,L-lactide) (PDLLA) and poly(lactide-co-glycolide) (PLGA).

In one aspect of the invention the amphiphilic block copolymer is a AB type diblock copolymer comprising
a poly(ethylene glycol) (PEG) or a monomethoxypoly(ethylene glycol) (mPEG) component (A) and
an amorphous hydrophobic biodegradable polymer component (B), which is poly(D,L-lactide) (PDLLA) or poly(lactide-co-glycolide) (PLGA).

In one preferred embodiment the hydrophilic component (A) is PEG or mPEG with a molecular weight of 1000 - 10000 Daltons and the hydrophobic polymer component (B) is PDLLA. The molecular weight of PDLLA can be calculated by the ratio of component B to component A which is in the range between 0.5 and 1.5

The block copolymer is represented by formula (I) 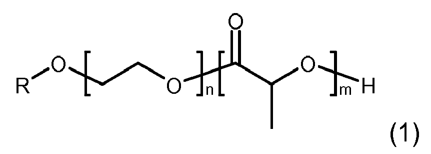 PEG-*b*-PDLLA or mPEG-b-PDLLA, wherein R is hydrogen or methyl; n is 22-114 and m is 7-104

In another aspect of the invention the amphiphilic block copolymer is a AB type diblock copolymer comprising
a monomethoxypoly(ethylene glycol) (mPEG) component (A) and
an amorphous hydrophobic biodegradable polymer component (B), which is poly(D,L-lactide) (PDLLA) n is 22-114 and m is 7-104

The ratio of the molecular weight of component B to component A is between 0.5 and 1.5, more preferably 1 and 1.2.

In one preferred embodiment the hydrophilic component (A) is mPEG with a molecular weight of 2000 Daltons and the hydrophobic polymer component (B) is PDLLA with a molecular weight of 2200 Daltons which is represented by formula (I) 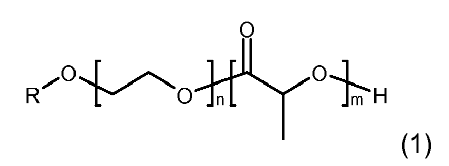 mPEG-b-PDLLA (2000-b-2200), wherein R is methyl; n is 45 and m is 30

In one embodiment of the present invention the ratio of the Molecular Weight of component B to component A is between 0.5 and 1.5, more preferably 1 and 1.2.

In further embodiments of the present invention, the epothilone is defined by the general formula (I) 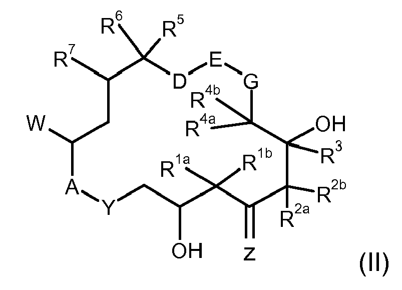 in which
- R^{1a}, R^{1b}: independently of one another are hydrogen, C₁-C₁₀-alkyl, aryl, aralkyl, or together are a group -(CH₂)ₘ-, where m is from 2 to 5;
- R^{2a}, R^{2b}: independently of one another are hydrogen, C₁-C₁₀-alkyl, C₂-C₁₀-alkenyl, C₂-C₁₀-alkynyl, aryl, aralkyl, or together are a group -(CH₂)ₙ-, where n is from 2 to 5,
- R³: is hydrogen, C₁-C₁₀-alkyl, aryl, aralkyl;
- R^{4a}, R^{4b}: independently of one another are hydrogen, C₁-C₁₀-alkyl, aryl, aralkyl, or together are a group -(CH₂)ₚ-, where p is from 2 to 5;
- R⁵: is hydrogen, C₁-C₁₀-alkyl, aryl, aralkyl, CO₂H, CO₂-al CH₂OH, CH₂O-C₁-C₅-alkyl, CH₂O-acyl, CN, CH₂NH₂, CH₂N((C₁-C₅-alkyl),acyl)_{1,2}, or CH₂Hal, CHal₃;
- R⁶, R⁷: independently of one another are hydrogen, or together are a further bond or an epoxide function;
- G: is O or CH₂;
- D-E: together are the group -H₂C-CH₂-, -HC=CH-, -C≡C-, -CH(OH)-CH(OH)-, -CH(OH)-CH₂-, -CH₂-CH(OH)-, -CH₂-O-, -O-CH₂-, or , where, if G is oxygen, D-E may not be CH₂-O; or
- D-E-G: together are the group H₂C-CH=CH;
- W: is the group C(=X)R⁸, or a bi- or tricyclic aromatic or heteroaromatic radical;
- X: is O or the group CR⁹R¹⁰;
- R⁸: is hydrogen, C₁-C₁₀-alkyl, aryl, aralkyl, halogen, CN;
- R⁹, R¹⁰: independently of one another are hydrogen, C₁-C₂₀-alkyl, aryl, aralkyl, or together with the methylene carbon atom are a 5- to 7-membered carbocyclic ring;
- Z: is O or hydrogen and the group OR¹¹;
- R¹¹: is hydrogen or a protective group PG^{z};
- A-Y: is a group O-C(=O), O-CH₂, CH₂-C(=O), NR¹²-C(=O), NR¹²-SO₂;
- R¹²: is hydrogen or C₁-C₁₀-alkyl;
- PG^{z}: is C₁-C₂₀-alkyl, a C₄-C₇-cycloa!ky! group which may contain one or more oxygen atoms in the ring, aryl, aralkyl, C₁-C₂₀-acyl, aroyl, C₁-C₂₀-alkylsulfonyl, arylsulfonyl, tri(C₁-C₂₀-alkyl)silyl, di(C₁-C₂₀-alkyl)arylsilyl, (C₁-C₂₀-alkyl)diarylsilyl or tri(aralkyl)silyl;

solubilized as individual stereoisomer or as a mixture of different stereoisomers and/or as a pharmaceutically acceptable salt.

For another embodiment of the present invention, the epothilone is defined by the formula (I)
in which
- R^{1a}, R^{1b}: independently of one another are hydrogen, C₁-C₄-alkyl, or together are the group -(CH₂)ₘ- where m is from 2 to 5;
- R^{2a}, R^{2b}: independently of one another are hydrogen, C₁-C₅-alkyl, or together are the group -(CH₂)ₘ-, where m is from 2 to 5, or C₂-C₆-alkenyl, or C₂-C₆-alkynyl;
- R³: is hydrogen;
- R^{4a}, R^{4b}: independently of one another are hydrogen, C₁-C₄-alkyl;
- R⁵: is hydrogen, C₁-C₄-alkyl, C(Hal)₃
- R⁶, R⁷: are both hydrogen, or together are a further bond, or together are an epoxide function;
- G: is CH₂;
- D-E: is the group H₂C-CH₂, or
- D-E-G: together are the group H₂C-CH=CH;
- W: is the group C(=X)R⁸, or a bi- or tricyclic aromatic or heteroaromatic radical;
- X: is the group CR⁹R¹⁰_{;}
- R⁸: is hydrogen, C₁-C₄-alkyl, halogen;
- R⁹, R¹⁰: are both independently of one another hydrogen, C₁-C₄-alkyl, aryl, aralkyl;
- Z: is oxygen;
- A-Y: is the group O-C(=O) or the group NR¹²-C(=O);
- R¹²: is hydrogen or C₁-C₄-alkyl ;
solubilized as an individual stereoisomer or as a mixture of different stereoisomers and/or as pharmaceutically acceptable salts.

In a further embodiment, what is solubilized are epothilones of the general formula (I) in which
- R^{1a}, R^{1b}: are both independently of one another hydrogen, C₁-C₂-alkyl, or together are a group -(CH₂)ₘ-, where m is from 2 to 5;
- R^{2a}, R^{2b}: are both independently of one another hydrogen, C₁-C₅-alkyl, or together are the group -(CH₂)ₙ-, where n is from 2 to 5, or C₂-C₆-alkenyl, or C₂-C₆-alkynyl;
- R³: is hydrogen;
- R^{4a}, R^{4b}: are both independently of one another hydrogen, C₁-C₂-alkyl;

- R⁵: is hydrogen or methyl or trifluoromethyl;
- R⁶, R⁷: together are a further bond, or together are an epoxide function;
- G: is CH₂;
- D-E: is the group H₂C-CH₂,
- D-E-G: together are the group H₂C-CH=CH;
- W: is the group C(=X)R⁸, or thiazolyl, oxazolyl, pyridyl, N-oxopyridyl, benzothiazolyl, benzoxazolyl, benzimidazolyl, which may optionally be substituted by C₁-C₃-alkyl, C₁-C₃-hydroxyalkyl, C₁-C₃-aminoalkyl, C₁-C₃-alkylsulfonyl;
- X: is the group CR⁹R¹⁰;
- R⁸: is hydrogen, methyl, chlorine, fluorine;
- R⁹, R¹⁰: are both independently of one another hydrogen, C₁-C₄-alkyl, thiazolyl, oxazolyl, pyridyl, N-oxopyridyl, which may optionally be substituted by C₁-C₃-alkyl, C₁-C₃-hydroxyalkyl, C₁-C₃-aminoalkyl, aralkyl;
- Z: is oxygen;
- A-Y: is the group O-C(=O) or the group NR¹²-C(=O);
- R¹²: is hydrogen or C₁-C₄-alkyl ;
as an individual stereoisomer or as a mixture of different stereoisomers and/or as a pharmaceutically acceptable salt.

In a further embodiment, what is solubilized are epothilones of the general formula (I) in which A-Y: O-C(=O); D-E: H₂C-CH₂; G: CH₂; Z: O; R^{1a}, R^{1b}: C₁-C₁₀-alkyl or together are a -(CH₂)ₚ- group, where p is from 2 to 3; R^{2a}, R^{2b} independently of one another are hydrogen, C₁-C₁₀-alkyl, C₂-C₁₀-alkenyl or C₂-C₁₀-alkynyl; R³ is hydrogen; R^{4a}, R^{4b} independently of one another are hydrogen or C₁-C₁₀-alkyl and R⁵ is C₁-C₁₀-alkyl.

In a further embodiment, what is solubilzed are epothilones of the general formula (I) in which R^{2a}, R^{2b} independently of one another are hydrogen, C₂-C₁₀-alkenyl orC₂-C₁₀-alkynyl; R⁶, R⁷ together are an epoxide function and W is a 2-methylbenzothiazol-5-yl group or a 2-methylbenzoxazol-5-yl group or a quinolin-7-yl group.

In a preferred embodiment, what is used in the polymeric composition are epothilones of the general formula (I) listed in the list below:
(4S,7R,8S,9S,13E/Z,16S)-4,8-dihyd roxy-16-(2-methylbenzoxazol-5-yl)-1-oxa-5,5,9,13-tetramethyl-7-(prop-2-en-1-yl)cyclohexadec-13-ene-2,6-dione;
(1S/R,3S,7S,10R,1R,12S,16R/S)-7,11-dihydroxy-10-(prop-2-en-1-yl)-3-(2-methylbenzoxazol-5-yl)-8,8,12,16-tetramethyl-4,17-dioxabicyclo-[14.1.0]heptadecane-5,9-dione;
(4S,7R,8S,9S,13E/Z,-16S)-4,8-dihydroxy-16-(2-methylbenzothiazol-5-yl)-1-oxa 5,5,9,13-tetramethyl-7-(prop-2-en-1-yl)cyclohexadec-13-ene-2,6-dione;
(1S/R,3S,7S,10R,1S,12S,16R/S)-7,11-dihydroxy-10-(prop-2-en-1-yl)-3-(2-methylbenzothiazol-5-yl)-8,8,12,16-tetramethyl-4,17-dioxabicyclo-[14.1.0]heptadecane-5,9-dione;
(1 S,3S,7S,10R,1S,12S,16R)-7,11-dihydroxy-10-(prop-2-en-1-yl)-3-(2-methylbenzothiazol-5-yl)-8,8,12,16-tetramethyl-4,17-dioxabicyclo[14.1.0]heptadecane-5,9-dione;
(1S/R,3S,7S,10R,11R,12S,16R/S)-7,11-dihydroxy-10-(prop-2-en-1-yl)-3-(2-methylbenzothiazol-5-yl)-8,8,12,16-tetramethyl-4,17-dioxabicyclo[14.1.0]-heptadecane-5,9-dione;
(4S,7R,8S,9S,13E/Z,-16S)-4,8-dihydroxy-16-(2-methylbenzothiazol-5-yl)-1-oxa 9,13-dimethyl-5,5-(1,3-thmethylene)-7-(prop-2-en-1-yl)cyclohexadec-13-ene-2,6-dione;
(1S/R,3S,7S,10R,11R,12S,16R/S)-7,11-dihydroxy-10-(prop-2-en-1-yl)-3-(2-methylbenzothiazol-5-yl)-12,16-dimethyl-8,8-(1,3-trimethylene)-4,17-dioxabicyclo[14.1.0]heptadecane-5,9-dione;
(4S,7R,8S,9S,13E/Z,-16S)-4,8-dihydroxy-16-(2-methylbenzothiazol-5-yl)-1-oxa 5,5,9,13-tetramethyl-7-(prop-2-yn-1-yl)cyclohexadec-13-ene-2,6-dione;
(1S/R,3S,7S,10R,11R,12S,16R/S)-7,11-dihydroxy-10-(prop-2-yn-1-yl)-3-(2-methylbenzothiazol-5-yl)-8,8,12,16-tetramethyl-4,17-dioxabicyclo-[14.1.0]heptadecane-5,9-dione;
(4S,7R,8S,9S,13E/Z,16S)-4,8-dihydroxy-16-(quinolin-7-yl)-1-oxa-5,5,9,13-tetramethyl-7-(prop-2-en-1-yl)cyclohexadec-13-ene-2,6-dione;
(1S/R,3S,7S,10R,11R,12S,16R/S)-7,11-dihydroxy-10-(prop-2-en-1-yl)-3-(quinolin-7-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(1,2-dimethyl-1H-benzimidazol-5-yl)-1-oxa-5,5,9,13-tetramethyl-7-(prop-2-en-1-yl)cyclohexadec-13-ene-2,6-dione;
(11S/R,3S,7S,10R,11R,12S,16R/S)-7,11-dihydroxy-10-(prop-2-en-1-yl)-3-(1,2-dimethyl-1H-benzimidazol-5-yl)-8,8,12,16-tetramethyl-4,17-dioxabicyclo[14.1.0]heptadecane-5,9-dione;
(4S,7R,8S,9S,13E/Z,-16S)-4,8-dihydroxy-16-(2-methylbenzothiazol-5-yl)-1-aza 5,5,9,13-tetramethyl-7-(prop-2-en-1-yl)cyclohexadec-13-ene-2,6-dione;
(1S/R,3S,7S,10R,11S,12S,16R/S)-7,11-dihydroxy-10-(prop-2-en-1-yl)-3-(2-methylbenzothiazol-5-yl)-8,8,12,16-tetramethyl-4-aza-17-oxabicyclo [14.1.0]heptadecane-5,9-dione,
(1S/R,3S,7S,10R,11R,12S,16R/S)-7,11-dihydroxy-10-(prop-2-en-1-yl)-3-(2-methylbenzothiazol-5-yl)-8,8,12,16-tetramethyl-4-aza-17-oxabicyclo-[14.1.0]heptadecane-5,9-dione,
(1S,3S(E),7S,10R,11S,12S,16R)-7,11-dihydroxy-8,8,10,12,16-pentamethyl-3-[1-(2-methyl-1,3-thiazol-4-yl)prop-1-en-2-yl]-4,17-dioxabicyclo[14.1.0]-heptadecane-5,9-dione,
(1S,3S(E),7S,10R,11S,12S,16R)-7,11-dihydroxy-8,8,10,12,16-pentamethyl-3-[1-(2-methyl-1,3-thiazol-4-yl)prop-1-en-2-yl]-17-oxa-4-azabicyclo[14.1.0]-heptadecane-5,9-dione,
(4S,7R,8S,9S,13Z,16S(E))-4,8-dihydroxy-5,5,7,9,13-pentamethyl-16-[1-(2-methyl-1,3-thiazol-4-yl)prop-1-en-2-yl]oxacyclohexadec-13-ene-2,6-dione,
(4S,7R,8S,9S,10E,13Z,16S(E))-4,8-dihydroxy-5,5,7,9,13-pentamethyl-16-[1-(2-methyl-1,3-thiazol-4-yl)prop-1-en-2-yl]oxacyclohexadec-10,13-diene-2,6-dione,
(4S,7R,8S,9S,10E,13Z,16S(E))-4,8-dihydroxy-5,5,7,9-tetramethyl-13-trifluoromethyl-16-[1-(2-methyl-1,3-thiazol-4-yl)prop-1-en-2-yl]oxacyclohexadec-10,13-diene-2,6-dione,
(1S,3S(E),7S,10R,11S,12S,16R)-7,11-dihydroxy-8,8,10,12,16-pentamethyl-3-[1-(2-methylsulfanyl-1,3-thiazol-4-yl)prop-1-en-2-yl]-4,17-dioxabicyclo[14.1.0]heptadecane-5,9-dione
as any individual stereoisomer or as a mixture of different stereoisomers and/or as a pharmaceutically acceptable salt.

Particular preference for the present composition is given to the epothilones (4S,7R,8S,9S,13E/Z,16S)-4,8-dihydroxy-16-(2-methylbenzothiazol-5-yl)-1-oxa-5,5,9,13-tetramethyl-7-(prop-2-en-1-yl)cyclohexadec-13-ene-2,6-dione;and (1S/R,3S,7S,10R,11S,16R/S)-7,11-dihydroxy-10-(prop-2-en-1-yl)-3-(2-methylbenzothiazol-5-yl)-8,8,12,16-tetramethyl-4,17-dioxabicyclo[14.1.0]-heptadecane-5,9-dione as any individual stereoisomer or as a mixture of different stereoisomers and/or as a pharmaceutically acceptable salt.

Most preferred is the polymeric composition comprising
(1S,3S,7S,10R,11S,12S,16R)-7,11-dihydroxy-10-(prop-2-en-1-yl)-3-(2-methylbenzothiazol-5-yl)-8,8,12,16-tetramethyl-4,17-dioxabicyclo[14.1.0]heptadecane-5,9-dione, as an individual stereoisomer or as a mixture of different stereoisomers and/or as a pharmaceutically acceptable salt.

### General Preparation

Amphiphilic block copolymers can be prepared according to the process as disclosed in US 5,702,717 or described in Reed, A.M. and Gilding, D.K. Polymer, 22 (1981), 494-498; Zhu et al., J. Appl. Polym.Sci. 39, (1990), 1-9; some are commercially available..

The polymeric composition of the present invention can be produced by the method consisting of the following steps:
(a) The amphiphilic block copolymer is weighed and dissolved in an organic solvent such as acetonitrile or acetone.
(b) Epothilone is dissolved in the same organic solvent
(c) The solutions according to a and b are mixed and the organic solvent is evaporated under reduced pressure at room temperature with subsequent drying at 0.1 mbar for 1 hour.
(d) Afterwards an inert gas is introduced allowing expulsion of the air and the containers are hermetically sealed immediately.

The composition obtained should be stored at temperatures ranging from 2° to 8°C.

The amount of epothilone within the composition and consequently used for process step (b) is an amount of less than 10% relating to the amount of the amphiphilic block copolymer, more preferable less than 8% and most preferred 5% and less.

Thus another aspect of the invention is the process for the preparation of the polymeric composition comprising an epothilone and an amphiphilic block copolymer having a hydrophilic poly(alkylene glycol) component (A) and an amorphous biodegradable hydrophobic polymer component (B).

More specifically a further aspect of the invention is the process for the preparation of the polymeric composition comprising an epothilone and an amphiphilic block copolymer having a hydrophilic poly(alkylene glycol) component (A) and an amorphous biodegradable hydrophobic polymer component (B) comprising the steps (a) - (d) as disclosed above.

A preferred aspect of the invention is the process for the preparation of the polymeric composition comprising an epothilone and an amphiphilic block copolymer as decribed in any of the embodiments above, especially the composition as disclosed in the examples.

In a subsequent step the the polymeric composition can be easily redispersed in an aqueous medium like water or pH adjusted solutions at physiological pH values for intravenous application resulting in a micellar dispersion. pH adjusted solutions can be e.g. a phosphate buffer solution, a phosphate buffered saline solution or saline solution. The pH can be adjusted to a range of 7 to 7.5.
The micellar dispersion obtained by dispersion of the product of the process described above effectively solubilizes the epothilone and can be injected into the body without further dilution or after further dilution with aqueous media. The latter is preferred.

Thus another aspect of the invention is a process for the production of the composition having the steps (a) - (d) mentioned above and additionally the step of redispersion. In a preferred aspect of the invention the redispersion is done in the clinic laboratory after sale of the product obtained by conducting the process steps (a) to (d).

For clinical purposes it is often convenient to serve a kit comprising the composition according to the invention together with means for redispersion.

Thus another aspect of the invention is a kit comprising the composition according to the invention together with means for redispersion.

Owing to the properties of the epothilones, which are suitable as therapeutics for tumor disorders or disorders associated with inflammatory reactions, the polymeric micelles according to the invention comprising an epothilone can be used for the treatment of tumor disorders or disorders associated with inflammatory reactions forms one aspect of the invention. A further aspect of the invention is a method for treating tumor disorders or disorders associated with inflammatory reactions where an effective amount of the active compound, comprised in a micellar system according to the invention, is administered to a human or an animal.

More specifically the micellar dispersion of the present invention can be used for the treatment of uterine carcinoma and cervical carcinoma, breast-, lung- and prostate carcinomas, renal cancer, malignant melanoma, bone metastases, brain tumors and brain metastases.

### Definitions

The term "aquous medium" includes water, saline, and pH adjusted solutions at physiological pH values for intravenous application resulting in a micellar dispersion.

"pH adjusted solutions" include e.g. a phosphate buffer solution, a phosphate buffered saline solution or saline solution.

The pH adjustment includes a range of 7 to 7,5.

The term "epothilone or epothilones" includes all natural epothilones and their derivatives. Epothilone derivatives are known, for example from WO 93/10102, WO 93/10121 and DE 41 38 042 A2, WO 97/19086 and WO 98/25929, WO 99/43320, WO 2000/066589, WO 00/49021, WO 00/71521, WO 2001027308, WO 99/02514, WO 2002080846.
The epothilones suitable for use in the present invention and their preparation are disclosed in DE 19907588, WO 98/25929, WO 99/58534, WO 99/2514, WO 99/67252, WO 99/67253, WO 99/7692, EP 99/4915, WO 00/1333, WO 00/66589, WO 00/49019, WO 00/49020, WO 00/49021, WO 00/71521, WO 00/37473, WO 00/57874, WO 01/92255, WO 01/81342, WO 01/73103, WO 01/64650, WO 01/70716, US 6204388, US 6387927, US 6380394, US 02/52028, US 02/58286, US 02/62030, WO 02/32844, WO 02/30356, WO 02/32844, WO 02/14323, and WO 02/8440. Particularly suitable epothilones are the compounds disclosed in WO 00/66589.
In the context of the present invention, preference is given to epothilones included in Formula I.
The term "epothilone" also includes the possibility that one or more epothilone derivatives selected from the disclosed list in one of the embodiments below are solubilized in a composition.

The term poly(lactide) includes poly(D- and poly(L-lactide) and mixtures such as e.g. poly(D,L-lactide) thereof.

The term "tumor disorders" encompasses the diseases or disease conditions associated with cellular growth, cell division and/or proliferation, such as, for example, malignant tumors. More specific disorders included in this term are, for example, osteoporosis, osteoarthritis, uterine carcinoma and cervical carcinoma, stomach cancer including all carcinomas of the upper abdominal region, intestinal cancer, adenocarcinomas, for example adenosarcoma, breast, lung (for example SCLC and NSCLC), head and neck carcinomas, malignant melanoma, acute lymphocytic or myelocytic leukemia, prostate carcinoma, bone metastases, brain tumors and brain metastases, where in this enumeration the terms "cancer" and "carcinoma" are used synonymously.
Solid tumors form a subaspect.
Preferably, "tumor disorders" are to be understood as meaning uterine carcinoma and cervical carcinoma, breast, lung and prostate carcinomas, malignant melanoma, bone metastases, brain tumors and brain metastases.

The term "pharmaceutical acceptable salts" includes addition salts of mineral acids, carbonic acids, sulfonic acids, e.g. salts of hydrochloric acid, hydrobromic acid, sulfuric acid, nitric acid, phosphoric acid, methanesulfonic acid, ethanesulfonic acid, toluolsulfonic acid, benzenesulfonic acid, naphthalinesulfonic acid, acetic acid, trifluoroacetic acid, propionic acid, lactic acid, tartaric acid, malic acid, citric acid, fumaric acid, pivalinic acid, maleic acid, succinic acid and benzoic acid.
In addition the term physiologically unobjectable salts includes salts of commonly suitable bases, e.g. salts of alkalimetall (e.g.. sodium- and potassium salts), alkaline earth salts (e.g. calcium- and magnesium salts) and ammonium salts, derivatized from NH₃ or organic amines with 1 to 16 carbon atoms, e.g. ethylamine, diethylamine, triethylamine, ethyldiisopropylamine, monoethanolamine, diethanolamine, triethanolamine, dicyclohexylamine, dimethylaminoethanol, prokaine, dibenzylamine, N-methylmorpholin, arginin, lysin, ethylendiamine and N-methylpiperidin.

The term "biodegradable polymer component" include homopolymers and copolymers. They are degraded enzymatically or non-enzymatically after injection into the human body and their non-toxic degradation products can be eliminated by metabolism or excretion.

The inert gas in the sense of the present invention can be e.g. argon, helium, nitrogen.

The "organic solvent" for use in process step (a) can be any solvent suitable to solve epothilones, e.g. acetone, acetonitrile.

In the sense of the invention the term "room temperature" includes temperatures in a range of 18° to 28°C.

The term "redispersion" means the addition of an aqueous medium to the composition to facilitate the formation of a micellar dispersion by dissolution of the polymeric composition into the liquid phase.

The term "micellar dispersion" describes micelles composed of the amphiphilic block copolymers with the hydrophobic component B forming the inner core and the hydrophilic component A forming the outer shell dispersed in an aquous medium. The micelle size is within a range of 1 to 100 nm, determined by Dynamic Light Scattering.

The term "alkyl" as used herein refers to straight-chain or branched-chain alkyl groups with 1-20 carbon atoms, e. g., methyl, ethyl, propyl, isopropyl, n-butyl, *t*-butyl, *n*-pentyl, isopentyl, neopentyl, heptyl, hexyl and decyl. Alkyl groups can be perfluorated or substituted by one to five substituents selected from the group consisting of halogen atoms, hydroxyl groups, C₁-C₄ alkoxy groups, or C₆-C₁₂ aryl groups (which can be substituted by one to three halogen atoms). If the term alkyl alone or in combination with other terms is used without giving any hint to the length of the chain the chain is meant to have at least one to five carbon atoms and the chain may be straight or branched and can optionally be substituted by hydroxy, halogen, methoxy.

The term "aryl" as used herein refer to a monocyclic aromatic carbocyclic e.g., phenyl, or heterocyclic ring moiety e.g. furyl, thienyl, pyridyl, pyrazolyl, pyrimidinyl, oxazolyl, pyridazinyl, pyrazinyl, thiazolyl, containing five to 14 ring atoms. Aryl groups can be substituted by one or more substituents selected from the group consisting of halogen, hydroxy, (C₁-C₁₀)alkoxy, -CO₂H -CO₂(C₁-C₁₀)alkyl, -NH₂, -NO₂, -N₃, -CN, (C₁-C₂₀)alkyl, (C₁-C₂₀)acyl, or (C₁-C₂₀)acyloxy. The heteroatoms can be oxidized, if as a result the aromatic character is not lost, e. g., a pyridine moiety can be oxidized to a pyridine N-oxide.or the term "aryl" refers to a bi- and tricyclic aryl radical, which can be a substituted or unsubstituted carbocyclic or heterocyclic radical with one or more heteroatoms, such as naphthyl, anthryl, benzoxazolyl, benzothiazolyl, benzimidazolyl, quinolyl, isoquinolyl, benzoxazinyl, benzofuranyl, indolyl, indazolyl, quinoxalinyl, tetrahydroisoquinolinyl, tetrahydroquinolinyl, thienopyridinyl, pyridopyridinyl, benzopyrazolyl, benzotriazolyl, or dihydroindolyl, which can be substituted in one or more places by halogen, OH, O-alkyl, CO₂H, CO₂-alkyl, -NH₂, -NO₂, -N₃, -CN, C₁-C₂₀-alkyl, C₁-C₂₀-acyl, or C₁-C₂₀-acyloxy groups, are suitable. The heteroatoms can be oxidized if as a result the aromatic character is not lost, such as, for example, the oxidation of a quinolyl to a quinolyl-N-oxide.

The term "aralkyl" as used herein refers to a group which can contain up to 14 atoms in the aryl ring (preferred five to ten) and one to eight carbon atoms in the alkyl chain (preferred one to four), e.g., benzyl, phenylethyl, naphthylmethyl, naphthylethyl, furylmethyl, thienylethyl, or pyridylpropyl. The rings can be substituted by one or more substituents selected from the group consisting of halogen, hydroxy, alkoxy, -CO₂H, -CO₂Alkyl, -NH₂, -NO₂, -N₃, -CN, C₁-C₂₀ alkyl, C₁-C₂₀ acyl, or C₁-C₂₀ acyloxy.

The term "alkenyl" as used herein refers to straight or branched alkenyl groups, e.g. Vinyl-, Allyl-, Homoallyl-, (*E*)-But-2-enyl-, (*Z*)-But-2-enyl-, (*E*)-But-1-enyl-, (*Z*)-But-1-enyl-, Pent-4-enyl-, (*E*)-Pent-3-enyl-, (*Z*)-Pent-3-enyl-, (*E*)-Pent-2-enyl-, (*Z*)-Pent-2-enyl-, (*E*)-Pent-1-enyl-, (*Z*)-Pent-1-enyl-, 2-Methylvinyl-, 3-Methylbut-3-enyl-, 2-Methylbut-3-enyl-, (*E*)-2-Methylbut-2-enyl-, (*Z*)-2-Methylbut-2-enyl-, 3-Methylbut-2-enyl-Gruppe.

The term "alkynyl" as used herein refers to straight or branched alkynyl groups, e.g. C≡C, -CH₂-C≡CH, -C≡C-CH₃, -CH(CH₃)-C≡CH, -C≡C-CH₂(CH₃), -C(CH₃)₂-C≡CH, -C≡C-CH(CH₃)₂-, -CH(CH₃)-C≡C-CH₃, -CH₂-C≡C-CH₂(CH₃).

The term "alkoxy" as used herein refers to C₁-C₁₀-alkoxy groups, preferably C₁-C₅-alkoxy groups, that can be straight-chain or branched e.g. methoxy, ethoxy, n-propoxy, iso-propoxy, n-butoxy, iso-butoxy, tert-butoxy or n-pentoxy, 2,2-dimethylpropoxy, 2-methylbutoxy or 3-methylbutoxy group. A methoxy or ethoxy group is preferred.

The term "acyl" means -C(O)alkyl or -C(O)phenyl or -C(O)benzyl, the alkyl chain may be straight or branched and has up to 12 carbon atoms and the phenyl ring may optionally be substituted by halogen, hydroxyl, C₁-C₅-alkoxy, and the acyl residue is derived from the known carbonic acids such as, e.g. CH(O)OH, CH₃-COOH, CH₃-CH₂-COOH, CH₃-CH₂-CH₂-COOH, CH₃-CH₂-CH₂-CH₂-COOH, pivalic acid.

The term "hal" or "halogen" means fluorine, chlorine, bromine or iodine, preferably in end products fluorine or chlorine, as a leaving group preferably iodine or bromine.

The residue CH₂N(alkyl, acyl)_{1,2} means that the nitrogen can bear one or two same or different residues selected from alkyl or acyl and both terms, alkyl and acyl have the meaning as defined above.

The protecting groups PG^{z} can be alkyl- and/or aryl-substituted silyl moieties, such as tris(C₁-C₂₀ alkyl)silyl, bis(C₁-C₂₀ alkyl)-arylsilyl, (C₁-C₂₀ alkyl)-diarylsilyl, tris(aralkyl)-silyl, C₁-C₂₀ alkyl, C₂-C₂₀-alkenyl, C₄-C₇ cycloalkyl, which may contain an oxygen atom in the ring, aryl, aralkyl, C₁-C₂₀ acyl, aroyl, C₁-C₂₀ alkoxycarbonyl C₁-C₂₀ alkylsulfonyl or C₁-C₂₀ arylsulfonyl. Protecting groups which can be easily be removed from the molecule are preferred, e.g., methoxymethyl, methoxyethyl, ethoxyethyl, tetrahydropyranyl, tetrahydrofuranyl, trimethylsilyl, triethylsilyl, triisopropylsilyl, *t*-butyldimethylsilyl, t-butyldiphenylsilyl, triphenylsilyl, tribenzylsilyl, benzyl, *p*-nitrobenzyl, *p-*methoxybenzyl, as well as alkylsulfonyl e.g. methylsulfonyl or arylsulfonyl e.g. p-tolylsulfonyl. As an alkoxycarbonyl radical, e.g., trichloroethyloxycarbonyl (Troc) is suitable. Preferred acyl groups are formyl, acetyl, propionyl, isopropionyl, trichloromethylcarbonyl pivaloyl, butyryl, or benzoyl, which all can be substituted by one or more amino and/or hydroxy moieties.

The etherified or esterified hydroxyl groups result e.g. in (C₁-C₅)-alkoxy or
(C₁-C₅)-alkoxy(C₁-C₅)alkyl groups e.g. MEM (Methylethoxymethyl), MOM (Methyloxymethyl), or cyclic ethers as e.g.THP (Tetrahydropyranylether), O(CO)(C₁-C₁₀)alkyl groups, O(CO)benzyl, O(CO)phenyl, with the used term alkyl as defined above.

As amino protective groups PG, the radicals that are known to one skilled in the art are considered. For example, the Alloc, Boc, Z, benzyl, f-Moc, Troc, Stabase or benzostabase group can be mentioned.

In one aspect of the invention all compounds defined in formula I as well as their salts, solvates and solvates of salts are encompassed,. especially the salts, solvates and salts of solvates of the compounds disclosed in the examples are one aspect of the invention as long as the disclosed compounds themselves are not already salts, solvates or solvates of the salts.

### Experimental Section:

The invention will now be described further in the examples given hereunder, without being limited to them.

### Abbreviations:

- PEG: Poly(ethylene glycol)
- mPEG: Monomethoxypoly(ethylene glycol)
- PDLLA: Poly(D,L-lactide)
- USP: United States Pharmacopoea

### Materials and Methods used:

mPEG-b-PDLLA (2000-b-2200) was purchased from Polymer Source Inc., Montreal, Canada.
Sagopilone was obtained from Bayer Schering Pharma AG, Germany. A method for its preparation is described in WO 00/66589.
Acetonitrile (Sigma Aldrich) was gradient grade
Phosphate Buffer Solution pH7,4 was prepared according to USP. Potassium dihydrogenphosphate and sodium hydroxide were obtained from Merck, Germany.

### Particle size and Polydispersity Index

The method for measuring the micelle particle size as well as the polydispersity index is done by Dynamic Light Scattering using a Zetasizer Nano from Malvern Instrument Ltd. (Worcestershire, UK) according to the following procedure: 1 to 2 ml of the micellar dispersion in a dust-free plastic cuvette is placed in the machine. The temperature is adjusted to 25°C for 1 minute. Briefly the principle is based on the measurement of the backscattered light fluctuations measured at an angle of 173° and the calculation of an autocorrelation function. The autocorrelation function is analysed using the DTS v5.1 software provided by Malvern to obtain the hydrodynamic diameter of the micelles (Zₐᵥₑ) stated as particle size herein and the particle size distribution (PDI) stated as polydispersity index herein. Measurements were done in triplicate for each probe and the mean values are calculated and used herein.

### Drug content and stability of sagopilone

The method for measuring the sagopilone content of the micellar dispersions as well as the detection of degradation products is determined by High Performance Liquid Chromatography. The analysis was done on a Agilent 1100 Series chromatography system consisting of a quaternary pump, an autoinjector, a column heater and a UV-detector from Agilent Technologies, Santa Clara, USA .

### HPLC conditions

| | |
|---|---|
| Column | 2 x Chromolith® Performance RP-18e 100x4,6 mm Merck, Germany |
| Mobile Phase | A - Acetonitrile |
| | B - Water |
| Pump Mode | Gradient |
| Temperature | 25°C |
| Injection Volume | 5 µl |
| Detection wavelength | 220 nm |

### Example 1: Preparation of polymeric compositions and solubilisation of sagopilone within micellar dispersions

4 Polymeric compositions of mPEG-b-PDLLA (2000-b-2200) and Sagopilone were prepared by four different methods. 1 Comparative composition was prepared without the block copolymer.

### Method A:

100 mg of mPEG-b-PDLLA (2000-b-2200) is weighed and dissolved in acetonitrile. 5 mg of Sagopilone is dissolved in the same organic solvent. The 2 solutions are mixed and the organic solvent is evaporated under reduced pressure at room temperature with subsequent drying at 0.1 mbar.

### Method B:

100 mg of mPEG-b-PDLLA (2000-b-2200) is weighed and dissolved in acetonitrile. 5 mg of Sagopilone is dissolved in the same organic solvent. The 2 solutions are mixed and the organic solvent is evaporated under stirring at room temperature.

### Method C:

100 mg of mPEG-b-PDLLA (2000-b-2200) is weighed and dissolved in acetonitrile. 5 mg of Sagopilone is dissolved in the same organic solvent. The 2 solutions are mixed and the organic solvent is evaporated under a stream of nitrogen at room temperature.

### Method D:

100 mg of mPEG-b-PDLLA (2000-b2200) and 5 mg of Sagopilone are weighed and mixed. The mixture is heated to 60°C in a water bath and stirred for 30 min. at this temperature. Afterwards the liquid composition is cooled to room temperature under stirring.

### Comparative Method E:

5 mg of Sagopilone is weighed and dissolved acetonitrile. The organic solvent is evaporated under reduced pressure at room temperature with subsequent drying at 0.1 mbar.

Subsequently 5 ml of phosphate buffer solution pH7.4 is added to each of the compositions. The resulting mixtures were shaken by hand to facilitate the formation of the micellar dispersion. The micellar dispersions were filtered using a 0.22 µm PVDF syringe filter. The filtrate was diluted immediately with an acetonitrile-water-mixture (1:1 v/v) and assayed by HPLC to determine the Sagopilone content.

**Table 1 Solubilisation efficiency and solubility of sagopilone within micellar dispersions after redispersion of the polymeric compositions**

| **Method** | **Solubilisation Efficiency [%]** | **Solubility of Sagopilone [mg/ml]** |
|---|---|---|
| **A** | **100** | **1,00** |
| **B** | **82** | **0,82** |
| **C** | **25** | **0,25** |
| **D** | **24** | **0,24** |
| **E** | **10** | **0,01** |

| | | |
|---|---|---|
| *Solubilisation Efficiency [%] = Solubilized drug [mg]/ added drug [mg]x100 | | |

The results shown in Table 1 indicate that the polymeric micellar dispersions increase the solubility of Sagopilone compared to the composition containing no block copolymer.
Regarding the industrial applicability of the polymeric composition a solubilisation efficiency of 100% is essential to ensure complete redispersion of the drug. Compositions prepared by Method C and D do not solubilize the drug sufficiently. Method B results in a significantly higher solubilization but still not enough. Only with the composition prepared by Method A the required concentration as well as 100% solubilisation efficiency within the micellar dispersion was achieved without loss of drug substance or degradation due to the process. Hence this clearly shows the superiority of this Method.

### Figure 1

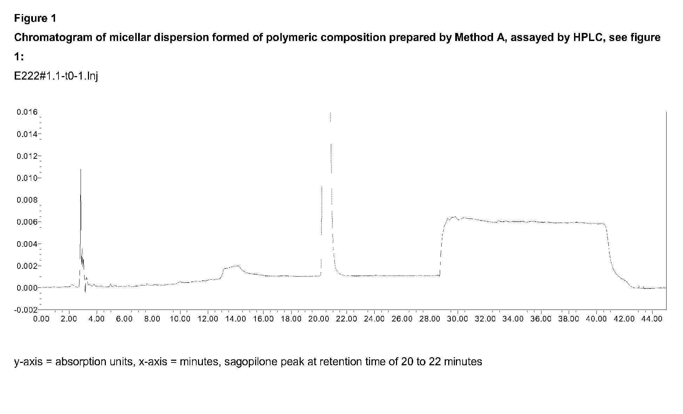

### Chromatogram of micellar dispersion formed of polymeric composition prepared by Method A, assayed by HPLC, see figure 1:

### Figure 2

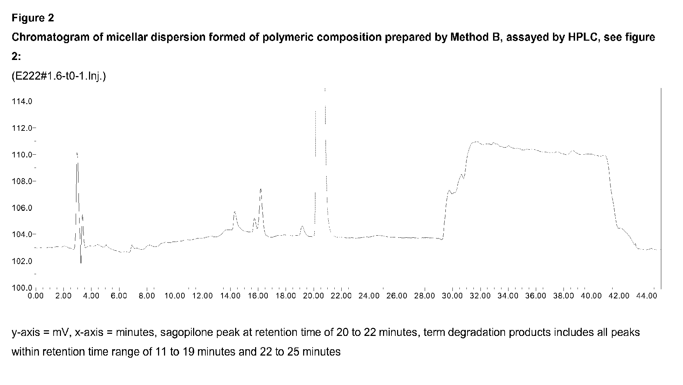

### Chromatogram of micellar dispersion formed of polymeric composition prepared by Method B, assayed by HPLC, see figure 2:

It can be derived from the chromatograms that degradation of the drug occurs during the preparation process of Method B indicated by degradation peaks within the retention time range of 11 to 17 minutes. In contrast no degradation is seen with Method A. This underlines the predominance of Method A, where the polymeric compositions are formed by evaporating the organic solvent under reduced pressure. Evaporation by stirring without reduced pressure as well as under the nitrogen stream already leads to degradation of the drug.

### Example 2: Solubilisation and stability of Sagopilone within micellar dispersions

3 Polymeric compositions of mPEG-b-PDLLA (2000-b-2200) and Sagopilone were prepared according to method A of Example 1.
Subsequently 5 ml of phosphate buffer solution pH7.4 is added to each of the compositions. The resulting mixtures were shaken by hand to facilitate the formation of the micellar dispersion. The micellar dispersions were filtered using a 0.22 µm PVDF syringe filter.
The obtained micellar dispersion was stored at 2 to 8 °C and aliquots were taken at certain time points. The Particle Size as well as the Polydispersity Index was determined. Afterwards the samples except for the one taken after preparation were filtered again through a 0.22 µm PVDF syringe filter to ensure the removal of precipitated drug substance. The filtrates were diluted immediately with an acetonitrile-water-mixture (1:1 v/v) and assayed by HPLC to determine the Sagopilone content.

**Table 2 Sagopilone content and particle size characterization of the micellar dispersions over 24 hours**

| | **Time point of analysis** | | |
|---|---|---|---|
| | **after preparation** | **after 6 hours** | **after 24 hours** |
| **Remained Sagopilone [%]** | **100** | **96** | **97** |
| **Particle Size [nm]** | **20** | **20** | **20** |
| **Polydispersity Index** | **0,01** | **0,01** | **0,01** |

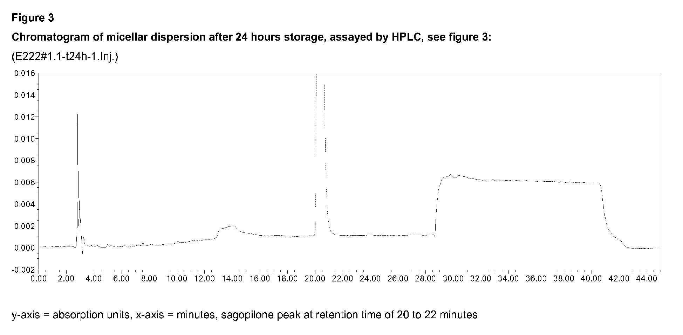

The polymeric compositions were easy and complete redispersable with 100 % solubilisation efficiency of Sagopilone. The resulting dispersions were clear. Precipitation of Sagopilone was not observed visually over 24 hours of storage. The remained drug analysed by HPLC did not change significantly. The micelles were stable in size and polydispersity index indicating that no bigger aggregates were present over this period of time. The Sagopilone itself was stable too, see figure 3. This is a very good premise for clinical application since the time interval between redispersion of the polymeric composition and its use should preferably be at least 12 hours.

### Figure 3

### Chromatogram of micellar dispersion after 24 hours storage, assayed by HPLC, see figure 3:

It can be derived from the chromatogram that Sagopilone solubilized within the polymeric micelles did not degrade over storage of 24 hours at 4°C.

### Example 3: Stability and redispersion behaviour of polymeric compositions over storage time and stability of the resulting micellar dispersions

Polymeric compositions were prepared by the method consisting of the following steps:
(a) 40 mg of mPEG-b-PDLLA (2000-b-2200) is weighed and dissolved in acetonitrile
(b) 2 mg of Sagopilone is dissolved in the same organic solvent
(c) The solutions according to a and b are mixed and the organic solvent is evaporated under reduced pressure at room temperature with subsequent drying at 0.1 mbar for 1 hour.
(d) Afterwards dry argon was introduced allowing expulsion of the air and the containers were hermetically sealed immediately.
   The solutions were stored at 2 to 8°C and 25 °C.
   After preparation, 1 and 3 months of storage the polymeric compositions were redispersed by the addition of 2 ml phosphate buffer solution pH7.4 and shaken by hand. Subsequently the dispersions were filtered through a 0.22 µm PVDF syringe filter. The redispersion behaviour, the Sagopilone content of the micellar dispersion and the stability of the dispersion regarding the Sagopilone content, particle size and polydispersity index (PDI) over 12 hours was analysed. For every storage condition and time point 6 individual polymeric compositions were analysed.

**Table 3**

| **Characterization of the micellar dispersions formed of polymeric compositions stored over 3 months at 6 and 25°C after preparation and 12 hours storage** | | | | | |
|---|---|---|---|---|---|
| | **storage [months]** | **Time [h] after Redispersion of Polymeric Composition** | | | |
| | | **0** | **12** | **0** | **12** |
| | | | | | |
| Sagopilone Content [%] | **0** | **100 ± 1** | **98 ± 2** | | |
| particle size | | 20 ± 0,1 | 20 ± 0,1 | | |
| PDI | | 0,01 | 0,01 | | |
| | | | | | |
| **Storage Temperature** | | **6°C** | | **25°C** | |
| | | | | | |
| Sagopilone Content [%] | **1** | **98 ± 3** | **95 ± 1** | **92 ± 5** | **30 ± 7** |
| particle size | | 20 ± 0,1 | 20 ± 0,1 | 21 ± 0,4 | 1656 ± 652 |
| PDI | | 0,01 | 0,01 | 0,05 | 0,91 |
| | | | | | |
| Sagopilone Content [%] | **3** | **96 ± 1** | **97 ± 2** | **57 ± 9** | **19 ± 3** |
| particle size | | 20 ± 0,1 | 20 ± 0,1 | 2056 ± 552 | n.d. |
| PDI | | 0,01 | 0,01 | 0,8 | n.d. |

In Table 3 the sagopilone content is the content of sagopilone within the dispersion in mg multiplied with 100 divided by the amount of sagopilone added during preparation in mg. The values present mean values and their standard deviation of 6 individual polymeric compositions of the respective parameters.

The redispersion behaviour of the compositions stored at 6 °C did not change over 3 months compared to those freshly prepared. All compositions were easily and complete redispersable. The resulting polymeric dispersions exhibit sufficient solubilization within a range of 100 ± 5 % and they are stable in drug concentration as well as particle size and polydispersity over 12 hours storage.

Polymeric compositions stored at 25°C were completey redispersable after 1 month whereas complete redispersion failed after 3 months of storage. Furthermore the dispersions formed after 1 month of storage were not stable indicated by the formation of a visible precipitate, particle sizes in a range of 1 to 3 micrometer with a very high polydispersity and a dramatically decreased sagopilone content.

The stability of the polymeric compositions as well as their excellent redispersion behaviour over the storage time of 3 months at 6°C indicates the feasibility of the use of the polymeric compositions according to this invention as stable formulations for sagopilone.
Thus an important condition for their industrial applicability is met.

### Figures

Figure 1
   Chromatogram of micellar dispersion formed of polymeric composition prepared by Method A, assayed by HPLC
Figure 2
   Chromatogram of micellar dispersion formed of polymeric composition prepared by Method B, assayed by HPLC
Figure 3
   Chromatogram of micellar dispersion after 24 hours storage, assayed by HPLC

## Claims

1. ) A stable polymeric composition comprising an amphiphilic AB type diblock copolymer having
a hydrophilic poly(alkylene glycol) component (A) and an amorphous biodegradable hydrophobic polymer component (B)
and
an epothilone.

2. ) A composition according to claim 1 in which the hydrophilic poly(alkylene glycol) component (A) is poly(ethylene glycol) (PEG) or monomethoxypoly(ethylene glycol) (mPEG) and the biodegradable hydrophobic polymer component (B) is poly(D,L-lactide) (PDLLA) or poly(lactide-co-glycol ide)(PLGA).

3. ) A composition according to claim 1 in which the hydrophilic poly(alkylene glycol) is poly(ethylene glycol) (PEG) component (A) has a polymerization degree of n=22-114 and the biodegradable hydrophobic poly(D,L-lactide) component (B) has a polymerization degree of m=7-104.

4. ) A composition according to claim 1 wherein the epothilone is an epothilone of formula (I) 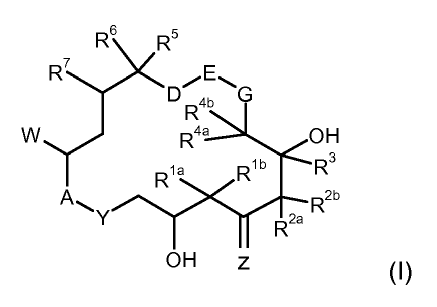 in which
R^{1a}, R^{1b} independently of one another are hydrogen, C₁-C₄-alkyl, or together are the group -(CH₂)ₘ- where m is from 2 to 5;
R^{2a}, R^{2b} independently of one another are hydrogen, C₁-C₅-alkyl, or together are the group -(CH₂)ₘ-, where m is from 2 to 5, or C₂-C₆-alkenyl, or C₂-C₆-alkynyl;
R³ is hydrogen;
R^{4a}, R^{4b} independently of one another are hydrogen, C₁-C₄-alkyl;
R⁵ is hydrogen, C₁-C₄-alkyl, C(Hal)₃
R⁶, R⁷ are both hydrogen, or together are a further bond, or together are an epoxide function;
G is CH₂;
D-E is the group H₂C-CH₂, or
D-E-G together are the group H₂C-CH=CH;
W is the group C(=X)R⁸, or a bi- or tricyclic aromatic or heteroaromatic radical;
X is the group CR⁹R¹⁰;
R⁸ is hydrogen, C₁-C₄-alkyl, halogen;
R⁹, R¹⁰ are both independently of one another hydrogen, C₁-C₄-alkyl, aryl, aralkyl;
Z is oxygen;
A-Y is the group O-C(=O) or the group NR¹²-C(=O);
R¹² is hydrogen or C₁-C₄-alkyl;
encapsulated as an individual stereoisomer or as a mixture of different stereoisomers and/or as pharmaceutically acceptable salts.

5. . A composition according to claim 1, in which the epothilone is selected from the group consisting of
(4S,7R,8S,9S,13E/Z,16S)-4,8-dihydroxy-16-(2-methylbenzoxazol-5-yl)-1-oxa-5,5,9,13-tetramethyl-7-(prop-2-en-1-yl)cyclohexadec-13-ene-2,6-dione;
(1S/R,3S,7S,10R,11R,12S,16R/S)-7,11-dihydroxy-10-(prop-2-en-1-yl)-3-(2-methylbenzoxazol-5-yl)-8,8,12,16-tetramethyl-4,17-dioxabicyclo-[14.1.0]heptadecane-5,9-dione;
(4S,7R,8S,9S,13E/Z,-16S)-4,8-dihydroxy-16-(2-methylbenzothiazol-5-yl)-1-oxa 5,5,9,13-tetramethyl-7-(prop-2-en-1-yl)cyclohexadec-13-ene-2,6-dione;
(1S/R,3S,7S,10R,11S,12S,16R/S)-7,11-dihydroxy-10-(prop-2-en-1-yl)-3-(2-methylbenzothiazol-5-yl)-8,8,12,16-tetramethyl-4,17-dioxabicyclo-[14.1.0]heptadecane-5,9-dione;
(1S,3S,7S,10R,11S,12S,16R)-7,11-dihydroxy-10-(prop-2-en-1-yl)-3-(2-methylbenzothiazol-5-yl)-8,8,12,16-tetramethyl-4,17-dioxabicyclo[14.1.0]heptadecane-5,9-dione;
(1S/R,3S,7S,110R,11R,12S,16R/S)-7,11-dihydroxy-10-(prop-2-en-1-yl)-3-(2-methylbenzothiazol-5-yl)-8,8,12,16-tetramethyl-4,17-dioxabicyclo[14.1.0]-heptadecane-5,9-dione;
(4S,7R,8S,9S,13E/Z,-16S)-4,8-dihydroxy-16-(2-methylbenzothiazol-5-yl)-1-oxa 9,13-dimethyl-5,5-(1,3-trimethylene)-7-(prop-2-en-1-yl)cyclohexadec-13-ene-2,6-dione;
(1S/R,3S,7S,110R,11R,12S,16R/S)-7,11-dihydroxy-10-(prop-2-en-1-yl)-3-(2-methylbenzothiazol-5-yl)-12,16-dimethyl-8,8-(1,3-trimethylene)-4,17-dioxabicyclo[14.1.0]heptadecane-5,9-dione;
(4S,7R,8S,9S,13E/Z,-16S)-4,8-dihydroxy-16-(2-methylbenzothiazol-5-yl)-1-oxa 5,5,9,13-tetramethyl-7-(prop-2-yn-1-yl)cyclohexadec-13-ene-2,6-dione;
(1S/R,3S,7S,10R,11R,12S,16R/S)-7,11-dihydroxy-10-(prop-2-yn-1-yl)-3-(2-methylbenzothiazol-5-yl)-8,8,12,16-tetramethyl-4,17-dioxabicyclo-[14.1.0]heptadecane-5,9-dione;
(4S,7R,8S,9S,13E/Z,16S)-4,8-dihydroxy-16-(quinolin-7-yl)-1-oxa-5,5,9,13-tetramethyl-7-(prop-2-en-1-yl)cyclohexadec-13-ene-2,6-dione;
(1S/R,3S,7S,10R,11R,12S,16R/S)-7,11-dihydroxy-10-(prop-2-en-1-yl)-3-(quinolin-7-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(1,2-dimethyl-1H-benzimidazol-5-yl)-1-oxa-5,5,9,13-tetramethyl-7-(prop-2-en-1-yl)cyclohexadec-13-ene-2,6-dione;
(1S/R,3S,7S,10R,11R,12S,16R/S)-7,11-dihydroxy-10-(prop-2-en-1-yl)-3-(1,2-dimethyl-1H-benzimidazol-5-yl)-8,8,12,16-tetramethyl-4,17-dioxabicyclo[14.1.0]heptadecane-5,9-dione;
(4S,7R,8S,9S,13E/Z,-16S)-4,8-dihydroxy-16-(2-methylbenzothiazol-5-yl)-1-aza 5,5,9,13-tetramethyl-7-(prop-2-en-1-yl)cyclohexadec-13-ene-2,6-dione;
(1S/R,3S,7S,10R,11S,12S,16R/S)-7,11-dihydroxy-10-(prop-2-en-1-yl)-3-(2-methylbenzothiazol-5-yl)-8,8,12,16-tetramethyl-4-aza-17-oxabicyclo [14.1.0]heptadecane-5,9-dione,
(1S/R,3S,7S,10R,11R,12S,16R/S)-7,11-dihydroxy-10-(prop-2-en-1-yl)-3-(2-methylbenzothiazol-5-yl)-8,8,12,16-tetramethyl-4-aza-17-oxabicyclo-[14.1.0]heptadecane-5,9-dione,
(1S,3S(E),7S,10R,11S,12S,16R)-7,11-dihydroxy-8,8,10,12,16-pentamethyl-3-[1-(2-methyl-1,3-thiazol-4-yl)prop-1-en-2-yl]-4,17-dioxabicyclo[14.1.0]-heptadecane-5,9-dione,
(1S,3S(E),7S,10R,11S,12S,16R)-7,11-dihydroxy-8,8,10,12,16-pentamethyl-3-[1-(2-methyl-1,3-thiazol-4-yl)prop-1-en-2-yl]-17-oxa-4-azabicyclo[14.1.0]-heptadecane-5,9-dione,
(4S,7R,8S,9S,13Z,16S(E))-4,8-dihydroxy-5,5,7,9,13-pentamethyl-16-[1-(2-methyl-1,3-thiazol-4-yl)prop-1-en-2-yl]oxacyclohexadec-13-ene-2,6-dione,
(4S,7R,8S,9S,10E,13Z,16S(E))-4,8-dihydroxy-5,5,7,9,13-pentamethyl-16-[1-(2-methyl-1,3-thiazol-4-yl)prop-1-en-2-yl]oxacyclohexadec-10,13-diene-2,6-dione,
(4S,7R,8S,9S,10E,13Z,16S(E))-4,8-dihydroxy-5,5,7,9-tetramethyl-13-trifluoromethyl-16-[1-(2-methyl-1,3-thiazol-4-yl)prop-1-en-2-yl]oxacyclohexadec-10,13-diene-2,6-dione,
(1S,3S(E),7S,10R,11S,12S,16R)-7,11-dihydroxy-8,8,10,12,16-pentamethyl-3-[1-(2-methylsulfanyl-1,3-thiazol-4-yl)prop-1-en-2-yl]-4,17-dioxabicyclo[14.1.0]heptadecane-5,9-dione
as an individual stereoisomer or as a mixture of different stereoisomers and/or as a pharmaceutically acceptable salt.

6. . A process for the preparation of the composition according to claim 1 comprising the following steps:
(a) The amphiphilic block copolymer is weighed and dissolved in an organic solvent.
(b) Epothilone, is dissolved in the same organic solvent
(c) The solutions according to (a) and (b) are mixed and the organic solvent is evaporated under reduced pressure at room temperature with subsequent drying at 0.1 mbar for 1 hour.
(d) Afterwards an inert gas is was introduced allowing expulsion of the air and the containers are hermetically sealed immediately.

7. . Use of the composition according to claim 1 for the preparation of a medicament for the treatment of tumor disorders or disorders associated with inflammatory reactions.

8. . A kit comprising the composition according to claim 1 together with means for redispersion.
